Europäisches Patentamt

European Patent Office

Office européen des brevets

(11) Veröffentlichungsnummer: **0 511 221 B1**

(12) **EUROPÄISCHE PATENTSCHRIFT**

(45) Veröffentlichungstag der Patentschrift: **14.09.94**

(51) Int. Cl.5: **C07K 3/24**, C12N 15/15

(21) Anmeldenummer: **91900759.1**

(22) Anmeldetag: **20.12.90**

(86) Internationale Anmeldenummer:
**PCT/EP90/02269**

(87) Internationale Veröffentlichungsnummer:
**WO 91/10677 (25.07.91 91/17)**

(54) **VERFAHREN ZUR ANREICHERUNG BZW. REINIGUNG VON HIRUDIN.**

(30) Priorität: **18.01.90 DE 4001238**

(43) Veröffentlichungstag der Anmeldung:
**04.11.92 Patentblatt 92/45**

(45) Bekanntmachung des Hinweises auf die
Patenterteilung:
**14.09.94 Patentblatt 94/37**

(84) Benannte Vertragsstaaten:
**AT BE CH DE DK FR GB IT LI NL SE**

(56) Entgegenhaltungen:

**Journal of Chromatography, Band 216, 1981, Elsevier Scientific Publishing Company, (Amsterdam, NL), V.Y. Ryashentsev et al."Behaviour of biomolecules in water-organic solvent-inorganic salt two-phase ternary systems", pages 346-349**

**R.K. Scopes "Protein purification", Zweite Auflage 1987, Springer-Verlag, (New York, US), S. 54-63**

**Bull. Soc. Chim. Biol., Band 45, Nr. 1, 1963, (Paris, FR), M. Jutisz et al., "Purification de l'hirudine", pages 55-67**

(73) Patentinhaber: **BASF Aktiengesellschaft
Carl-Bosch-Strasse 38
D-67063 Ludwigshafen (DE)**

(72) Erfinder: **LANG, Kurt
Birkenstrasse 159
D-8122 Penzberg (DE)**
Erfinder: **Kreimeyer, Andreas
Uhlandstrasse 5
W-6725 Roemerberg (DE)**

## Beschreibung

Effektive und kostengünstige Reinigungsverfahren spielen bei der Herstellung chemischer und biologischer Produkte eine zentrale Rolle. Im Zuge der Entwicklung der Bio- und Gentechnologie in den letzten Jahren hat insbesondere die Verfahrensentwicklung zur Reinigung von Proteinen, Peptiden und Vitaminen im technischen Maßstab zunehmend Bedeutung erlangt.

Ein wichtiger Schritt bei der Isolierung der meisten zellulären Inhaltsstoffe ist die Fraktionierung des Ausgangsmaterials oder einer Zwischenstufe im Verlauf der Reinigung. Hierzu werden in der Regel (fraktionierte) Fällungen oder Verteilungschromatographien angewendet. Zur Durchführung von Fällungen werden die Eigenschaften des Lösungsmittels durch die Zugabe entsprechender Reagenzien derart verändert, daß die Löslichkeit des Proteins stark abnimmt und dieses ausfällt. Die Löslichkeit kann durch die Zugabe von Salzen wie Ammoniumsulfat oder organischen Lösungsmitteln wie Ethanol oder durch eine Veränderung des pH-Wertes oder der Temperatur variiert werden, (Green, A.A. & Hughes W.L. in "Methods in Enzymology", Band I, S. 67-90 (1955); Belter et al, 1988, Seite 100ff. in "Bioseperations", Hrsg.: Belter, P.A., Cussler, E.L. & Hu, W.-S.).

Auch Flüssig-Flüssig-Zwei-Phasen Verteilungschromatographien werden seit langem zur Reinigung von Biomolekülen eingesetzt. In den 40er und 50er Jahren wurden ausschließlich organisch-wäßrige Zwei-Phasen-Systeme mit nicht mit Wasser mischbaren organischen Lösungsmitteln verwendet. Diese sind zwar zur Anreicherung und Reinigung niedermolekularer Biomoleküle wie Antibiotika und Vitamine sehr geeignet (Schlügerl, K., 1987, in "Separations for Biotechnology", Hrsg.: Verral, M.S. & Hudson, M.J. S. 260-269), ihrem Einsatz bei der Isolierung insbesondere von Proteinen sind jedoch enge Grenzen gesetzt. Insbesondere die Denaturierung und Präzipitation der Proteine in den organisch-wäßrigen Systemen bereiten große Probleme (Johansson G., 1985, in "Partitioning in aqueous two-phase systems", Acad. Press, S. 161-225).

Eine andere Methode zur Flüssig-Flüssig-Zwei-Phasen-Extraktion wurde Ende der 50er Jahre von Albertson entwickelt. Er verwendete 2-Phasen-Systeme mit zwei wäßrigen Phasen und konnte zeigen, daß diese insbesondere zur Anreicherung von Proteinen sehr geeignet sind (Nature 182, 709 (1958)). Zur Extraktion von Proteinen mit 2-Phasen-Systemen wurden seitdem fast ausschließlich wäßrige 2-Phasen-Systeme verwendet und weiterentwickelt.

Aufgrund der hohen Einsatzstoffkosten für die phaseninduzierenden Polymere wie Polyethylenglykol oder Dextran werden wäßrige 2-Phasen-Extraktionen im technischen Maßstab jedoch nur selten durchgeführt.

In J. Chromatography 216, 346 (1981) wird eine Methode zur Reinigung von Biomolekülen beschrieben, die darin besteht, daß man das rohe Protein in Wasser löst und anschließend Natriumchlorid und n-Propanol zufügt. Versucht man, nach der Lehre dieser Literatur Hirudin zu reinigen, so stellt man fest, daß man mit den genannten Lösungsmittel-Wasser-Mischungen Hirudin nicht anreichern kann.

Gegenstand der Erfindung ist ein Verfahren zur Anreicherung von Hirudin, dadurch gekennzeichnet, daß man das Hirudin-haltige Gemisch in einer Mischung aus Wasser und 1-Propanol, 2-Propanol, n-Butanol oder Aceton bei einem pH-Wert von 4,0 bis 4,3 aufnimmt, das Gemisch mit Natriumchlorid versetzt und die dabei entstehende Unterphase mit dem Hirudin abtrennt.

Zur Durchführung des Verfahrens wird das Hirudin zunächst in Wasser oder einen Puffer und/oder unmittelbar in ein wäßrig-organisches Lösungsmittel-System gebracht.

Bei der Zugabe des organischen Lösungsmittels zu der wäßrigen Phase muß darauf geachtet werden, daß das Hirudin in Lösung bleibt.
Falls das Hirudin nicht in Lösung bleibt, empfiehlt es sich, den pH-Wert, die Temperatur, den Anteil und die Art des organischen Lösungsmittels, die Art des Puffers und dessen Konzentration, die Hirudinkonzentration und die Art und Konzentration des Salzes zu variieren. Fällt bei der Zugabe des organischen Lösungsmittel eine andere Substanz aus, wird diese abzentrifugiert oder abfiltriert. Als Lösungsmittel eignen sich am besten n-Butanol und 2-Propanol.

Zu der so erhaltenen Lösung wird zur Phasentrennung das Natriumchlorid zugegeben. Die am besten geeignete Menge wird in einem Vorversuch ermittelt. In der Regel ist die Phasentrennung am besten, wenn so viel Salz zugegeben wird, daß die Phasen mit dem Salz gesättigt sind. Bei der Wahl der richtigen Parameter ist auch darauf zu achten, daß das Hirudin nach der Phasentrennung ganz überwiegend in der wäßrigen Phase vorliegt. Für die Wahl der Parameter läßt sich keine allgemeine Regel aufstellen, es empfiehlt sich, die Parameter in einigen Vorversuchen zu ermitteln.

Tritt bei der Phasentrennung ein Niederschlag auf, so wird dieser verworfen.

Aus der wäßrigen Phase wird das Hirudin in bekannter Weise beispielsweise durch hydrophobe Chromatographie oder Fällung isoliert. Es kann auch weiteren Reinigungsschritten unterworfen werden.

Das erfindungsgemäße Verfahren ist schnell, kostengünstig und einfach durchführbar. Es zeichnet sich gegenüber dem bisherigen organisch-wäßrigen Extraktionen dadurch aus, daß das zu reinigende Hirudin in der organischwäßrigen Mischphase gelöst bleibt und erst durch die anschließende Phasentrennung bevorzugt in die sich abtrennende wäßrige Phase übergeführt wird. Ein besonderer Vorteil des neuen Verfahrens ist es, daß das Hirudin sich aufgrund der Verwendung von mit Wasser mischbaren organischen Lösungsmitteln ständig in einem wäßrigen Milieu befinden.

Beispiel 1

Organisch-wäßrige Verteilungschromatographie mit Hirudin-haltigem Zellextrakt

Es wurde von einem grob vorgereinigten Zellextrakt aus E. coli ausgegangen, der neben Hirudin noch zahlreiche Fremdproteine, Peptide sowie teilweise gefärbte Bestandteile des Fermentationsmediums und der Zellen enthielt. Hirudin war zu etwa 10 % angereichert (bezogen auf das Protein).

Eine wäßrige Lösung dieses Extraktes mit einer Proteinkonzentration von 10 - 100 mg/ml wurde bei 0 - 20°C mit 1 Volumen i-Propanol versetzt und der pH-Wert der Mischphase mit 2N HCl auf pH 1 - 2 eingestellt. Ausgefallenes Material wurde durch Zentrifugation (30 min, 10000 g, 0 - 20°C) oder Filtration abgetrennt. Der überstand bzw. das Filtrat wurde anschließend mit 2N NaOH auf pH 4 - 4,3 eingestellt und unter Rühren mit festen NaCl bis zur Sättigung versetzt. Nach Beendigung des Rührvorganges setzte eine spontane Trennung der organischen und wäßrigen Phasen ein. Zugleich bildete sich zwischen der organischen und der wäßrigen Phase eine flächige massive Interphase überwiegend aus ausgefallenen Proteinen. Das Hirudin befand sich fast ausschließlich in der wäßrigen Phase. Die Ausbeute an aktivem Hirudin in der wäßrigen Phase betrug 70 - 90 %. Eine weitere Ausbeuteerhöhung ist möglich, wenn die organische Phase wiederholt mit $H_2O$/Kochsalz extrahiert wird.

Durch die Extraktion wurde das Hirudin um mindestens den Faktor 5 angereichert. Weiterhin wurden (teilweise gefärbte) hydrophobe Verunreinigung in die i-Propanolphase überführt, die nach der Phasentrennung eine stark dunkle Färbung aufwies.

Beispiel 2

Organisch-wäßrige Verteilungschromatographie mit Hirudin und verschiedenen Phasensystemen

Zur Durchführung der Verteilungschromatographien mit Hirudin mit verschiedenen Lösungssystemen und Salzen wurde von einer wäßrigen Hirudinlösung der Konzentration 1 mg/ml in 20 mM Natriumacetat, pH 4,0 ausgegangen.

Mit mehreren Kombinationen von verschiedenen Lösungsmitteln und Salzen konnte das Hirudin stark überwiegend in der wäßrigen Phase angereichert werden (vgl. Tab. ).

**Tabelle**

Organisch-wäßrige Verteilungschromatographie mit Hirudin in verschiedenen Lösungsmittelsystemen und Salzen

| Salzart | Lösungsmittel System (v/v = 50/50) | Volumenanteil nach Phasentrennung (%) | | Aktivitätsverteilung nach Phasentrennung % | |
|---|---|---|---|---|---|
| | | untere Phase (H₂O) | obere organ. Phase | untere Phase (H₂O) | obere organ. Phase |
| NaCl | 2-Propanol/H₂O | 48 | 52 | > 90 | < 10 |
| | Aceton/H₂O | 75 | 25 | > 90 | < 10 |
| | 1-Propanol/H₂O | 48 | 52 | > 80 | < 10 |

**Patentansprüche**

1. Verfahren zur Anreicherung von Hirudin, dadurch gekennzeichnet, daß man das Hirudin- haltige Gemisch in einer Mischung aus Wasser und 1-Propanol, 2-Propanol, n-Butanol oder Aceton bei einem pH-Wert von 4,0 bis 4,3 aufnimmt, das Gemisch mit Natriumchlorid versetzt und die dabei entstehende Unterphase mit dem Hirudin abtrennt.

**Claims**

1. A method for concentrating hirudin, which comprises taking up the hirudin-containing mixture in a mixture of water and 1-propanol, 2-propanol, n-butanol or acetone at a pH of 4.0-4.3, adding sodium chloride to the mixture, and separating off the resulting lower phase with the hirudin.

**Revendications**

1. Procédé d'enrichissement d'hirudine, caractérisé par le fait que l'on reprend le mélange contenant l'hirudine dans un mélange d'eau et de 1-propanol, 2-propanol, n-butanol ou acétone, à un pH de 4,0 à 4,3, on additionne le mélange de chlorure de sodium et on sépare la sous-phase ainsi produite avec l'hirudine.